# EUROPÄISCHE PATENTSCHRIFT

(11) **EP 1 832 658 B1**
(45) Veröffentlichungstag und Bekanntmachung des Hinweises auf die Patenterteilung: **06.07.2011**
(21) Anmeldenummer: 07103215.5
(22) Anmeldetag: 28.02.2007
(51) Int. Cl.: C12P 7/02

(54) **Verfahren zur Herstellung von optisch aktiven sekundären Alkoholen aus Ketonen unter Verwendung einer adsorbierten Carbonylreduktase-Aktivität**
Method for producing optically acitve secondary alcohols from ketones employing an adsorbed carbonyl reductase activity
Procédé de préparation d'un alcool secondaire optiquement actif à partir d'une cétone en utilisant l'activité d'une carbonyl réductase adsorbée

(30) Priorität: 09.03.2006 DE 102006010994
(43) Veröffentlichungstag der Anmeldung: 12.09.2007
(73) Patentinhaber: Wacker Chemie AG, 81737 München (DE)
(72) Erfinder: Pfaller, Rupert, 80639, München (DE); Stohrer, Jürgen, 82049, Pullach (DE)
(74) Vertreter: Potten, Holger

(56) Entgegenhaltungen:
- EP-A1- 1 568 780
- WO-A-2005/121326
- WO-A-2006/061137
- NAKAMURA, K. ET AL.: "Asymmetric reduction of ketones by Geotrichum candidum in the presence of Amberlite XAD, a solid organic solvent" JOURNAL OF THE CHEMICAL SOCIETY, PERKIN TRANSACTIONS 1, Nr. 19, 2000, Seiten 3205-3211, XP001203859

## Beschreibung

Die Erfindung betrifft ein Verfahren zur effizienten enzymatischen Herstellung chiraler Alkohole.

Optisch aktive Hydroxyverbindungen sind wertvolle Synthesebausteine, z. B. bei der Herstellung pharmazeutischer Wirkstoffe oder von Agrochemikalien. Diese Verbindungen sind durch klassische chemische Verfahren oft nur schwer herstellbar, denn die erforderlichen optischen Reinheiten für Anwendungen im pharmazeutischen oder agrochemischen Bereich sind auf diesem Wege nur schwer zu erreichen. Daher werden zur Herstellung chiraler Verbindungen im zunehmenden Maße biotechnologische Verfahren angewendet. Speziell Enzyme, die Carbonylverbindungen reduzieren können, finden wegen ihrer hohen Enantioselektivität vermehrt Verwendung.

Enzyme aus der Klasse der Oxidoreduktasen, die zur Herstellung chiraler Verbindungen durch Reduktion prochiraler Carbonylverbindungen eingesetzt werden, werden mit dem Sammelbegriff Carbonylreduktase (in der Folge "CR") bezeichnet. In der Mehrzahl der Fälle ist das Produkt einer CR-Reaktion ein Alkohol. Es ist aber auch möglich, dass das Produkt einer CR-Reaktion ein Amin ist. Zu den Carbonylreduktasen zählen unter anderem Alkoholdehydrogenasen (in der Folge "ADH"), Aldo-Ketoreduktasen ("AKR"), Aldehydreduktasen, Glycerindehydrogenasen und die Fettsäuresynthase (bezeichnet als "FAS"). Aber auch Aminotransferasen oder Aminosäuredehydrogenasen (z. B. Threonindehydrogenase) sind zu den Carbonylreduktasen zu zählen. Diesem breiten Spektrum reduzierender Enzyme ist gemeinsam, dass sie die Elektronen für die Reduktion der Carbonylverbindung aus Redox-Cofaktoren in deren reduzierter Form, üblicherweise NADH oder NADPH, beziehen.

Die Redox-Cofaktoren NADH bzw. NADPH werden bei der enzymatischen Reduktion stöchiometrisch verbraucht, d.h. sie müssen entweder stöchiometrisch eingesetzt werden oder aber durch Oxidation eines Cosubstrats regeneriert werden (sog. Cofaktor-Regenerierung). Ein Cosubstrat ist dabei definiert als eine Verbindung, die als Reduktionsmittel enzymatisch oxidiert wird, wobei die dabei gewonnenen Elektronen auf NAD bzw. NADP übertragen werden und somit NADH bzw. NADPH regeneriert wird.

Diese Biotransformationsverfahren haben in den meisten Fällen den Nachteil einer geringen Wirtschaftlichkeit, indem der Enzymeinsatz sehr hoch ist und die Raum-Zeitausbeuten niedrig. Darüber hinaus wird die wirtschaftliche Effizienz einer Biotransformation oft dadurch beeinträchtigt, dass entweder das Edukt oder das Produkt zur Inaktivierung des Enzyms führt.

Aus Nakamura, K. et al., "Asymmetric reduction of ketones by Geotrichum candidum in the presence of Amberlite XAD, a solid organic solvent", Journal of the Chemical Society, Perkin Transactions 1, Nr. 19, 2000, Seiten 3205-3211 ist eine Biotransformationszusammensetzung bekannt, umfassend Fermenterzellen, enthaltend ein Carbonylreduktase-Enzym, ein Adsorbens (die Verbindung Amberlite XAD) und ein Keton.

Einen Überblick über industriell genutzte Biotransfornationen gibt Breuer et al. (2004), Angew. Chem. 116: 806-843.

Beides, Enzymeinsatz und Raum-Zeitausbeute, sind entscheidende Kostenfaktoren, welche die Wirtschaftlichkeit einer Biotransformation bestimmen. Der Stand der Technik stellt derzeit keine allgemein einsetzbaren Verfahren zur Verfügung, mit denen chirale Alkohole auf kostengünstige Weise hergestellt werden können.

Um einen effizienten Enzymeinsatz zu gewährleisten, ist es prinzipiell möglich, das Enzym aus einem Biotransformationsansatz wiederzugewinnen, indem der Reaktionsansatz extrahiert wird, wobei das Enzym in der wässrigen Reaktionsphase verbleibt. Voraussetzung dafür ist jedoch eine hohe Stabilität des Enzyms gegenüber organischen Lösungsmitteln. EP 1568780 offenbart eine Methode zur Rückgewinnung und zum Wiedereinsatz des Enzyms LB-ADH bei der Reduktion von Acetessigsäuremethylester zu (R)-3-Hydroxybuttersäuremethylester, allerdings bei niedrigem Edukteinsatz von 5 % (w/v).

Die Erhöhung der Raum-Zeitausbeute ist eine andere Möglichkeit zur Verbesserung der Wirtschaftlichkeit von Biotransformationen. DE102005038606 (Bsp. 3) offenbart die Herstellung von (R)-1-Acetoxy-2-Propanol aus Acetoxyaceton bei einer hohen Eduktdosierung von 43 % (w/v), jedoch bei hohem Enzymeinsatz ohne Möglichkeit der Enzymrückgewinnung. Zur Verbesserung der Wirtschaftlichkeit von Biotransformationen wäre ein Verfahren wünschenswert, das hohe Raum-Zeitausbeuten mit einem effizienten Enzymeinsatz kombiniert.

Adsorbentien finden breite Anwendung bei biotechnologischen Verfahren. Sie werden unter anderem eingesetzt als Filtrationshilfen, um Schwebestoffe zu entfernen. Sie werden weiterhin in der Chromatographie zur Stofftrennung eingesetzt und finden auch als Trägermaterialien Verwendung.

In der enzymatischen Synthese ist der Einsatz von Trägermaterialien vor allem zur Immobilisierung von Enzymen bekannt. Beschrieben wurde auch die Immobilisierung ganzer Zellen in Alginat und die Beeinflussung der Enantioselektivität in Gegenwart eines Adsorbens. Das bekannteste Beispiel eines Trägermaterials zur Immobilisierung von Enzymen ist Eupergit© der Fa. Röhm. Kommerziell erhältlich sind vor allem kovalent immobilisierte Lipasen (z. B. erhältlich von der Fa. Novozymes), die zur Racematspaltung eingesetzt werden können. Die Herstellung immobilisierter Enzyme ist allerdings ein aufwändiger Prozess, so dass deren Einsatz üblicherweise nicht mit einer signifikanten Kostenersparnis verbunden ist. Kovalent immobilisierte Enzyme sind also nicht geeignet, die Wirtschaftlichkeit der Synthese chiraler Alkohole zu verbessern.

Bisher ist somit kein einfaches und kostengünstiges Verfahren bekannt, dass es dem Fachmann ermöglicht, unter Einsatz von Adsorbentien die Wirtschaftlichkeit der Herstellung chiraler Alkohole durch enzymatische Reduktion bezüglich Enzymeinsatz und Raum-Zeitausbeute zu verbessern.

Aufgabe der Erfindung war es, ein Verfahren zur Verfügung zu stellen, welches eine effiziente kostengünstige Herstellung eines chiralen sekundären Alkohols ermöglicht.

Diese Aufgabe wird gelöst durch ein Verfahren, bei dem eine Biotransformationszusammensetzung, enthaltend ein Keton der Formel (I), 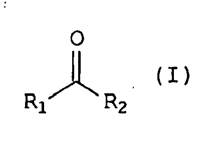 wobei R₁ und R₂ verschieden sind und jeweils organischer Rest bedeuten, eine Oxidoreduktase und ein Cosubstrat, zur Reaktion gebracht wird, wobei ein chiraler sekundärer Alkohol entsteht, dadurch gekennzeichnet, dass die Biotransformationszusammensetzung ein Adsorbens, ausgewählt aus der Gruppe Aluminiumoxid, Aluminiumoxidhydrat, Siliziumdioxid, Kieselsäure, gefällte Kieselsäure, pyrogene Kieselsäure, Silikat, Calciumphosphat, kationische und anionische Ionenaustauscher auf Basis der Polystyrolharze, Polysaccharid, vernetztes Dextran, vernetzte Agarose, Aktivkohle, Methacrylat, enthält, welches mit der Oxidoreduktase assoziiert und welches nach dem Ende der Reaktion von der Biotransformationszusammensetzung abgetrennt wird und die mit Adsorbens assoziierte Oxidoreduktase für den Wiedereinsatz vom Reaktionsgemisch durch Filtration, Sedimentation oder Zentrifugation abgetrennt wird.

Mittels des erfindungsgemäßen Verfahrens wird die Oxidoreduktase aus der Biotransformationszusammensetzung zurückgewonnen und kann erneut für die Biotransformation eines Ketons der Formel (I) in einen chiralen sekundären Alkohol eingesetzt werden. Das erfindungsgemäße Verfahren ermöglicht es, auch bei hohen Raum-Zeitausbeuten den Enzymeinsatz bei Biotransformationen durch Recycling zu minimieren.

Je nach Oberflächenbeschaffenheit können diese Adsorbentien für hydrophobe oder elektrostatische Interaktion geeignet sein.

Die geeigneten Adsorbentien finden sich in den Produktkatalogen einschlägiger Hersteller wie z. B. Merck, Fluka, Röhm, Rohm und Haas, Sigma-Aldrich, Supelco (Produktkatalog "Chromatographie: Produkte für die Analytik und Aufreinigung", 2006-2007, S. 561-602) oder GE Healthcare (Produktkatalog "Products for Life Sciences", 2006, S. 506-636).

Bevorzugte Adsorbentien sind XAD, Florisil^{®}, Dowex^{®}, Amberlite^{®}, Kieselgel, Celite^{®}.

Besonders bevorzugtes Adsorbens ist Celite®.

Die genannten Adsorbentien finden zwar bereits die im Stand der Technik dargelegte Anwendung bei biotechnologischen Verfahren, diese Anwendungen liefern allerdings keinen Hinweis auf das erfindungsgemäße Verfahren. Insbesondere war es völlig unerwartet, dass es durch den Einsatz der Adsorbentien gelingt, das funktionelle Enzym aus der Biotransformationszusammensetzung zurück zu gewinnen, obwohl diese Zusammensetzungen einen für enzymatische Reaktionen ungewöhnlich hohen Anteil organischer Verbindungen von 50 - 80 % (v/v) (zusammengesetzt aus Edukt, Cosubstrat und eventuell Lösemittel) enthalten.

Vorzugsweise handelt es sich bei dem chiralen sekundären Alkohol um eine Verbindung der Formel (II) oder (III) 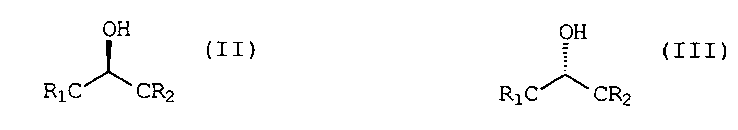 wobei R₁ und R₂ voneinander verschieden sind und organischer Rest bedeuten.

Vorzugsweise sind R₁ und R₂ verschiedene organische Reste mit 1-20 C-Atomen, wie z.B. unverzweigte oder verzweigte C₁-C₂₀-Alkyl-, C₂-C₂₀-Alkenyl-, C₂-C₂₀-Alkinyl-, C₃-C₈-Cycloalkyl-, C₆-C₂₀-Aryl- oder C₅-C₂₀-Heteroarylreste, wobei ein oder mehrere C-Atome der Reste R₁ oder R₂ durch Atome, ausgewählt aus der Gruppe B, N, O, Si, P und S, ersetzt sein können oder durch F, Cl, Br, J, durch ggf. substituierte C₃-C₈-Cycloalkyl, C₆-C₂₀-Aryl, C₅-C₂₀-Heteroaryl, durch Silylreste sowie durch CN, NH₂, NO oder NO₂ substituiert sein können.

Besonders sind R₁ und R₂ ausgewählt aus der Gruppe C₁-C₁₂-Alkyl-, C₁-C₁₂-Alkenyl-, C₂-C₁₂-Alkinyl-, C₃-C₈-Cycloalkyl-, C₆-C₂₀-Aryl- oder C₅-C₂₀-Heteroarylrest, bei dem ein oder mehrere C-Atome durch F, Cl, C₃-C₈-Cycloalkyl, C₆-C₂₀-Aryl oder C₅-C₂₀-Heteroaryl substituiert sein können oder durch Atome, ausgewählt aus der Gruppe N, O und S, ersetzt sein können.

Bei der Oxidoreduktase handelt es sich vorzugsweise um eine CR mit S- oder R-Spezifität.

Als R-spezifische CRs werden vorzugsweise sekundäre ADHs, z. B. aus Stämmen der Gattung Lactobacillus wie die ADHs aus Lactobacillus brevis (LB-ADH), Lactobacillus kefir, Lactobacillus parabuchneri, Lactobacillus kandleri, Lactobacillus minor, oder es werden Fettsäuresynthetasen (FAS), besonders bevorzugt die FAS der Bäckerhefe oder aus Pichia pastoris eingesetzt. Bevorzugte R-selektive CRs sind ADHs der Gattung Lactobacillus. Besonders bevorzugte R-selektive CR ist die LB-ADH.

Als S-spezifische CRs werden vorzugsweise sekundäre ADHs, z. B. aus Stämmen der Gattung Thermoanaerobacter (Thermoanaerobium) wie die ADHs aus Thermoanaerobacter sp. (T-ADH, offenbart in DE 102004029112 A1), Th. brockii oder Th. ethanolicus, oder aus Stämmen der Gattung Rhodococcus wie die ADHs aus Rhodococcus ruber (RR-ADH) oder Rhodococcus erythropolis (RE-ADH), oder es werden CRs aus der Bäckerhefe (Beispiele S-spezifischer CRs aus der Bäckerhefe sind in Kaluzna et al. (2004), J. Am. Chem. Soc. 126: 12827-12832 offenbart) eingesetzt. Bevorzugte S-selektive CRs sind ADHs der Gattungen Thermoanaerobacter und Rhodococcus. Besonders bevorzugte S-selektive CRs sind die T-ADH und die RR-ADH. Insbesondere bevorzugte S-selektive CR ist die T-ADH.

Die zur enzymatischen Reduktion verwendeten CRs können hergestellt werden, indem man den Mikroorganismus kultiviert, aus dem die betreffende CR stammt. Dies geschieht jeweils in einer dem Fachmann bekannten Art und Weise. Das auf diese Weise hergestellte CR Enzym kann direkt in den Zellen des Produktionswirts verwendet werden, es kann aber auch nach Aufschluss der Zellen als Proteinextrakt, bzw. nach entsprechender Aufarbeitung durch z. B. Säulenchromatographie als gereinigtes Protein eingesetzt werden. Die Enzymproduktion der CRs kann mit einem sog. Expressionssystem auch in rekombinanter Form erfolgen. Dazu wird das für die betreffende CR kodierende Gen isoliert und, dem Stand der Technik entsprechend, in einen für die Proteinproduktion geeigneten Expressionsvektor kloniert. Nach Transformation des Expressionsvektors in einen geeigneten Wirtsorganismus wird ein Produktionsstamm isoliert. Mit diesem Produktionsstamm lässt sich die CR in an sich bekannter Weise, z. B. durch Fermentation, produzieren. Das auf diese Weise hergestellte CR Enzym kann dann direkt in den Zellen des Produktionswirts weiterverwendet werden oder aber nach Aufschluss der Zellen als Proteinextrakt, bzw. nach entsprechender Aufarbeitung durch z. B. Säulenchromatographie als gereinigtes Protein. Bevorzugt ist die Enzymproduktion der erfindungsgemäßen CRs mit einem Expressionssystem in rekombinanter Form. Für die Enzymproduktion sind bakterielle und eukaryontische Expressionssysteme geeignet. Wirtsorganismen für die Enzymproduktion sind vorzugsweise ausgewählt aus Escherichia coli, Stämmen der Gattung Bacillus, Hefen wie Pichia pastoris, Hansenula polymorpha oder Saccharomyces cerevisiae sowie Pilzen wie Aspergillus oder Neurospora, sie sind aber nicht auf die genannten Wirtsorganismen beschränkt. Zu den bevorzugten Expressionssystemen zählen E. coli, Bacillus, Pichia pastoris, S. cerevisiae, Hansenula polymorpha oder Aspergillus. Besonders bevorzugte Expressionssysteme für die Produktion des CR Enzyms sind E. coli, Pichia pastoris und S. cerevisiae. Insbesondere bevorzugtes Expressionssystem ist E. coli.

Um einen möglichst kosteneffizienten Enzymeinsatz zu erreichen erfolgt die Enzymproduktion vorzugsweise durch Fermentation, besonders bevorzugt in einem Fed Batch Verfahren.

Vorzugsweise werden die Zellen aus der Fermentation (Fermenterzellen) dann direkt, im Fermentationsmedium suspendiert oder nach vorheriger Isolierung und anschließender Resuspension, in dem erfindungsgemäßen Verfahren weiterverwendet, so dass das erfindungsgemäße Verfahren als Ganzzellbiotransformation geführt wird. Es ist jedoch auch möglich, im erfindungsgemäßen Verfahren nach Aufschluss der Zellen den so erhaltenen Proteinextrakt, oder nach entsprechender Aufarbeitung durch z. B. Säulenchromatographie das so erhaltene gereinigte Protein im erfindungsgemäßen Verfahren einzusetzen.

Besonders bevorzugt ist die Ganzzellbiotransformation, bei der zuerst die Enzymproduktion in einer rekombinanten Wirtszelle mittels Fermentation erfolgt und die Fermenterzellen anschließend direkt, im Fermentationsmedium suspendiert oder nach vorheriger Isolierung und anschließender Resuspension, in einer erfindungsgemäßen Biotransformation verwendet werden.

Ggf. umfasst die Biotransformationszusammensetzung einen Redox-Cofaktor. Bei dem Redox-Cofaktor handelt es sich um eine Verbindung, die in ihrer reduzierten Form Elektronen zur Verfügung stellt, die in der enzymatischen Reaktion durch eine Oxidoreduktase auf das Edukt übertragen werden mit dem Resultat, dass ein erfindungsgemäßes Produkt entsteht. Der Redox-Cofaktor ist vorzugsweise ausgewählt aus Verbindungen der Gruppe NAD, NADP, (jeweils oxidierte Form des Cofaktors), NADH, NADPH (jeweils reduzierte Form des Cofaktors) und deren Salzen.

Die Redox-Cofaktoren in ihrer reduzierten Form, NADH bzw. NADPH, werden bei der enzymatischen Reduktion stöchiometrisch verbraucht, d.h. sie müssen entweder stöchiometrisch eingesetzt werden oder aber durch Oxidation eines Cosubstrats regeneriert werden (Cofaktor-Regenerierung). Der stöchiometrische Einsatz von NADH oder NADPH ist aufgrund des hohen Preises dieser Verbindungen unwirtschaftlich. Dieser Nachteil wird durch die Cofaktor-Regenerierung umgangen. Voraussetzung dafür sind ein billiges Cosubstrat (Reduktionsmittel) und ein Cofaktorreduzierendes Enzym. Erst die effiziente und billige Regenerierung des Redox-Cofaktors ermöglicht den technischen Einsatz biokatalytischer Reduktionsverfahren.

Der Einsatz eines Adsorbens erlaubt die einfache Abtrennung des Enzyms aus dem Reaktionsansatz durch z. B. Filtration, Sedimentation oder Zentrifugation und seinen Wiedereinsatz in einer neuen Biotransformationszusammensetzung, wobei ev. bei der Rückgewinnung verlorenes Enzym im neuen Ansatz aufgestockt werden kann, um die maximale Umsatzrate beizubehalten. Auf diese Weise ist es möglich, eine prinzipiell unbeschränkte Zahl von Reaktionszyklen durchzuführen.

Ein Cosubstrat ist eine Verbindung, die als Reduktionsmittel enzymatisch oxidiert wird, wobei die dabei gewonnenen Elektronen auf NAD bzw. NADP übertragen werden und somit NADH bzw. NADPH regeneriert wird.

Wenn im erfindungsgemäßen Verfahren eine CR aus der Klasse der ADHs eingesetzt wird, so wird als Cosubstrat für die Cofaktor-Regenerierung ein Alkohol, vorzugsweise ein billiger Alkohol wie Isopropanol oder 2-Butanol eingesetzt. Es eignen sich aber auch alle anderen, vom 2-Butanol abgeleiteten höheren sekundären Alkohole. Damit wird in dieser Verfahrensvariante sowohl die stereoselektive Reduktion des Eduktes als auch die Cofaktor-Regenerierung vom gleichen Enzym, der ADH besorgt.

Wenn im erfindungsgemäßen Verfahren eine CR, die nicht eine ADH ist, eingesetzt wird, so erfolgt die Cofaktor-Regenerierung mittels eines zweiten Enzyms. Dieses befindet sich ebenfalls im Reaktionsansatz. Die CR reduziert das Edukt stereoselektiv zum gewünschten Produkt wobei der Cofaktor NADH bzw. NADPH verbraucht wird. Die Regenerierung des verbrauchten NADH bzw. NADPH wird durch ein zweites Enzym bewerkstelligt. Prinzipiell ist jedes Enzym zur Cofaktor-Regenerierung geeignet, das in einer enzymatischen Reaktion ein Substrat oxidiert und gleichzeitig NAD zu NADH bzw. NADP zu NADPH reduziert. Vorzugsweise wird ein Enzym verwendet, welches ein möglichst billiges Cosubstrat wie beispielsweise Glucose oder Ameisensäure, bzw. dessen Salze, oxidiert. Vorzugsweise wird als Enzym zur Cofaktor-Regenerierung ein Enzym aus der Gruppe Glucose-Dehydrogenase (GDH) und Formiat-Dehydrogenase (FDH) eingesetzt.

Bevorzugte Kombinationen von Enzym/Cosubstrat zur Cofaktorregenerierung sind die Kombination einer ADH mit einem Alkohol wie z.B. Isopropanol oder 2-Butanol oder die Kombination einer GDH mit Glucose.

Besonders bevorzugt ist die Kombination einer ADH mit einem alkohol wie z,B. Isopropanol oder 2-Butanol.

Insbesondere bevorzugt ist die Kombination einer ADH mit Isopropanol.

Das erfindungsgemäße Verfahren ermöglicht es durch enzymatische Reduktion eines Eduktes der Formel (I) bei hohen Raum-Zeitausbeuten und einem geringen Enzymeinsatz chirale sekundäre Alkohole mittels eines einfachen Batch-Verfahrens herzustellen.

In der einfachsten Form umfasst eine Biotransformationszusammensetzung (sog. Batchansatz) Fermenterzellen enthaltend ein CR-Enzym, ein genanntes Adsorbens, als Edukt eine Verbindung der Formel (I), einen Redox-Cofaktor ausgewählt aus den Verbindungen NAD, NADH, NADP, NADPH, und deren Salzen, ein Cosubstrat ausgewählt aus der Gruppe Isopropanol, 2-Butanol und Glucose sowie bei Verwendung von Glucose als Cosubstrat eine GDH als Cofaktor-regenerierendes Enzym.

In einer abgewandelten Form des Verfahrens werden eine oder mehrere der genannten Komponenten der Biotransformationszusammensetzung kontinuierlich oder diskontinuierlich zudosiert. (sog. Fed Batchansatz).

Bevorzugtes Verfahren ist der Batchansatz.

Das im erfindungsgemäßen Verfahren eingesetzte CR-Enzym kann in ganzen Zellen enthalten sein (Ganzzellverfahren) oder aber nach Aufschluss der Zellen als Proteinextrakt, bzw. nach entsprechender Aufarbeitung durch z. B. Säulenchromatographie ale gereinigtes Protein in der Biotransformationszusammensetzung eingesetzt werden.

Bevorzugt ist der Einsatz des CR-Enzyms in einem Ganzzellverfahren.

Vorzugsweise enthält eine Biotransformationszusammensetzung zwischen 1 % (v/v) und 40 % (v/v) einer aus der Fermentation erhaltenen Suspension von Fermenterzellen, mit einem Biomassenteil von 0,05 - 2 % (w/v), enthaltend ein CR-Enzym. Der Biomasseanteil ist dabei definiert als Trockenbiomasse, die man erhält, wenn die Fermenterzellen, z. B. in einem Trockenschrank bei 105°C, bis zur Gewichtskonstanz getrocknet werden.

Besonders bevorzugt enthält die Zusammensetzung zwischen 5 % (v/v) und 30 % (v/v) einer aus der Fermentation erhaltenen Suspension von Fermenterzellen, enthaltend ein CR-Enzym, mit einem Biomasseanteil von 0,25 - 1,5 % (w/v).

Insbesondere bevorzugt enthält die Zusammensetzung wischen 10 % (v/v) und 25 % (v/v) einer aus der Fermentation erhaltenen Suspension von Fermenterzellen, enthaltend ein CR-Enzym, mit einem Biomasseanteil von 0,5 - 1,25 % (w/v).

Eine Biotransformationszusammensetzung enthält außerdem zwischen 0,1 % (w/v) und 10 % (w/v) eines der genannten Adsorbentien.

Bevorzugt enthält die Zusammensetzung zwischen 0,2 % (w/v) und 5 % (w/v) eines der genannten Adsorbentien.

Besonders bevorzugt enthält die Zusammensetzung zwischen 0,5 % (w/v) und 3 % (w/v) eines der genannten Adsorbentien.

Der Einsatz des Adsorbens erlaubt die einfache Abtrennung des Enzyms vom Reaktionsansatz durch z. B. Filtration, Sedimentation oder Zentrifugation und seinen wiedereinsatz in einer neuen Biotransformationszusammensetzung, wobei eventuell bei der Rückgewinnung verlorenes Enzym im neuen Ansatz aufgestockt werden kann, um die maximale Umsatzrate beizubehalten. Auf diese Weise ist es prinzipiell möglich, eine unbeschränkte Zahl von Reaktionszyklen durchzuführen. Bevorzugt ist ein Verfahren, bei dem bis zu 20 Reaktionszyklen durchgeführt werden.

Besonders bevorzugt ist ein Verfahren, bei dem bis zu. 4 Reaktionszyklen durchgeführt werden.

Das CR-Enzym kann dabei in ganzen Zellen enthalten oder aber nach Aufschluss der Zellen als Proteinextrakt, bzw. nach entsprechender Aufarbeitung durch z. B. Säulenchromatographie als gereinigtes Protein in der Biotransformationszusammensetzung eingesetzt werden.

Eine Biotransformationszusammensetzung zeichnet sich ferner dadurch aus, dass der Anteil an Edukt der Formel (I) vorzugsweise zwischen 5 % (w/v) und 60 % (w/v) des Gesamtansatzes beträgt.

Bevorzugt liegt der Anteil an Edukt der Formel (I) zwischen 20 % (w/v) und 50 % (w/v) des Gesamtansatzes.

Insbesondere bevorzugt ist eine Zusammensetzung, bei der der Anteil an Edukt der Formel (I) zwischen 30 % (w/v) und 45 % (w/v) des Gesamtansatzes beträgt.

Eine Biotransformationszusammensetzung zeichnet sich auch dadurch aus, dass der Anteil an Cosubstrat im Falle von Isopropanol oder 2-Butanol vorzugsweise zwischen 10 % (w/v) und 50 % (w/v) des Gesamtansatzes beträgt.

Besonders bevorzugt beträgt der Anteil an Cosubstrat im Falle von Isopropanol oder 2-Butanol zwischen 20 % (w/v) und 45 % (w/v) des Gesamtansatzes.

Insbesondere bevorzugt beträgt der Anteil an Cosubstrat im Falle von Isopropanol oder 2-Butanol zwischen 30 % (w/v) und 40 % (w/v) des Gesamtansatzes.

Wird Glucose als Cosubstrat verwendet, enthält die Zusammensetzung Glucose vorzugsweise in einer Konzentration von 20 % (w/v) bis 65 % (w/v) bezogen auf den Gesamtansatz.

Eine Biotransformationszusammensetzung umfasst vorzugsweise den Redox-Cofaktor in einer Konzentration zwischen 10 µM und 200 µM, besonders bevorzugt zwischen 20 µM und 150 µm, insbesondere bevorzugt zwischen 40 µM und 100 µM.

Das erfindungsgemäße Verfahren wird vorzugsweise bei einer Temperatur von 3°C bis 70°C, bevorzugt von 5°C bis 50°C, insbesondere bevorzugt von 15°C bis 40°C durchgeführt.

Vorzugsweise wird es in einem pH-Bereich von 5 bis 9, bevorzugt von 5,5 bis 8, insbesondere bevorzugt von 6 bis 7,5 durchgeführt. Vorzugsweise ist der Ansatz zur Konstanthaltung des pH Wertes gepuffert. Vorzugsweise erfolgt eine pH-Kontrolle über eine Titrationsvorrichtung, gekoppelt an ein pH-Messgerät (sog. pH-Stat Methode).

Das erfindungsgemäße Verfahren wird vorzugsweise bei einem Druck von 1 mbar bis 2 bar, bevorzugt bei Normaldruck durchgeführt.

Das erfindungsgemäße Verfahren kann außerdem in Gegenwart einer weiteren organischen Phase durchgeführt werden.

Die Reaktionsdauer des erfindungsgemäßen Verfahrens beträgt vorzugsweise 5 h bis 100 h, besonders bevorzugt 10 h bis 60 h, insbesondere bevorzugt 15 h bis 40 h.

Unter den genannten Bedingungen werden Edukte der allgemeinen Formel (I) zu > 80 %, bevorzugt > 90 %, insbesondere bevorzugt > 93 % zu einem chiralen sekundären Alkohol umgesetzt.

Das erfindungsgemäße Verfahren ermöglichte es erstmals, aus Edukten der allgemeinen Formel (I) bei hoher Raum-Zeitausbeute (Eduktdosierung >20 % w/v, 24 h Reaktionsdauer) unter Rückgewinnung des Enzyms (mindestens viermaliges Recycling) einen chiralen sekundären Alkohol mit Ausbeuten >90 % und einem ee >99 % herzustellen, wobei gleichzeitig die Dosierung CRhaltiger Zellen, ausgedrückt in Trockenbiomasse Fermenterzellen, nicht mehr als 1 % (w/v) vom Ansatzvolumen betrug. Die beobachtete Effizienz bei gleichzeitiger Möglichkeit der Enzymrückgewinnung und die für eine Biotransformation unerwartet hohen Raum-Zeitausbeuten des erfindungsgemäßen Biotransformationsverfahrens waren nach dem Stand der Technik nicht zu erwarten. Insbesondere ist überraschend, dass es auch in einer wässrig-alkoholischen Lösung gelingt, den Grossteil der eingesetzten Alkoholdehydrogenase adsorptiv und mit uneingeschränkter Aktivität aus der Reaktionslösung abzutrennen. Überraschend ist auch, dass bereits die Gegenwart des Adsorbens genügt, um die Enzymaktivität während der Umsetzung zu stabilisieren.

Die Isolierung des Produktes erfolgt nach an sich bekannten Methoden, wobei vorzugsweise zuerst das mit dem Adsorbens assoziierte Enzym für den Wiedereinsatz vom Reaktionsgemisch abgetrennt wird, vorzugsweise durch Filtration, Sedimentation oder Zentrifugation.

Aus dem verbleibenden Reaktionsansatz kann das Produkt dann direkt isoliert werden, vorzugsweise durch Destillation oder durch Extraktion mit einem mit Wasser nicht mischbaren, organischen Lösungsmittel.

Bei der direkten Destillation erhält man das gewünschte Endprodukt. Das Endprodukt wird typischerweise in einer Ausbeute >70 %, bevorzugt >80 %, besonders bevorzugt >90 %, jeweils bezogen auf die eingesetzte Menge des Eduktes (I), erhalten. Es besitzt einen Enantiomerenüberschuss von bevorzugt ee >90 %, besonders bevorzugt ee >97 % insbesondere bevorzugt ee = 100 %.

Die alternative Isolierung des Produktes aus dem Reaktionsgemisch durch Extraktion kann diskontinuierlich (batchweise) oder kontinuierlich erfolgen. Wie dem Fachmann bekannt, wird dabei eine geeignete Temperatur eingestellt, so dass eine optimale Extraktion des Produktes aus der wässrigen Phase gewährleistet ist. Vorzugsweise erfolgt die Extraktion bei einer Temperatur von 10 bis 70°C.

Als organische Lösemittel sind alle mit Wasser nicht mischbaren Lösemittel geeignet, die eine Verbindung der Formel (II) oder (III) aus einer wässrigen Phase extrahieren können.

Bevorzugt werden organische Lösemittel, ausgewählt aus der Gruppe der Ester, Ether, Alkane, Aromaten und chlorierten Kohlenwasserstoffe verwendet.

Besonders bevorzugt werden Ethylacetat, Methylacetat, Propylacetat, Isopropylacetat, Butylacetat, Tertbutylacetat, Diethylether, Diisopropylether, Dibutylether und Methyl-Tertbutylether (MTBE), Pentan, Hexan, Heptan, Toluol, Methylenchlorid, Chloroform oder deren Mischungen verwendet.

Insbesondere bevorzugte Lösungsmittel sind MTBE, Ethylacetat, Butylacetat und Methylenchlorid.

Nach der Abtrennung der organischen Extraktionsphase wird diese vorzugsweise destillativ aufgearbeitet, wobei eine Anreicherung des Reaktionsproduktes erreicht und die teilweise bis vollständige Abtrennung von Nebenprodukten vom Extraktionslösemittel bewirkt wird und dieses erneut zur Extraktion eingesetzt werden kann.

Durch Aufreinigung der organischen Extraktionslösung enthaltend das Rohprodukt, beispielsweise mittels Feindestillation, erhält man das gewünschte Endprodukt. Das Endprodukt wird typischerweise in einer Ausbeute >70 %, bevorzugt >80 %, besonders bevorzugt >90 %, jeweils bezogen auf die eingesetzte Menge des Eduktes (I), erhalten. Es besitzt einen Enantiomerenüberschuss von bevorzugt ee >90 %, besonders bevorzugt ee >97 %, insbesondere bevorzugt ee = 100 %.

Die folgenden Beispiele dienen zur Beschreibung der Erfindung:

### 1. Beispiel:

### Herstellung von Carbonylreduktasen durch Fermentation

Das Enzym LB-ADH, sein Gen und die rekombinante Produktion von LB-ADH in E. coli sind in EP796914 offenbart. Es wurde das in E. coli transformierte und in EP796914 offenbarte Plasmid pADH-1 verwendet. Alternativ kann das Enzym als aus rekombinanten E. coli hergestellter Rohextrakt kommerziell von der Fa. Jülich Chiral Solutions GmbH bezogen werden.

Das Enzym T-ADH, sein Gen und die rekombinante Produktion von T-ADH in E. coli ist offenbart in DE 102004029112 A1. Es wurde das in E. coli transformierte und in DE 102004029112 A1 offenbarte Plasmid pET24a [ADH-TS] verwendet. Alternativ kann das Enzym als aus rekombinanten E. coli hergestellter Rohextrakt kommerziell von der Fa. Jülich Chiral Solutions GmbH bezogen werden.

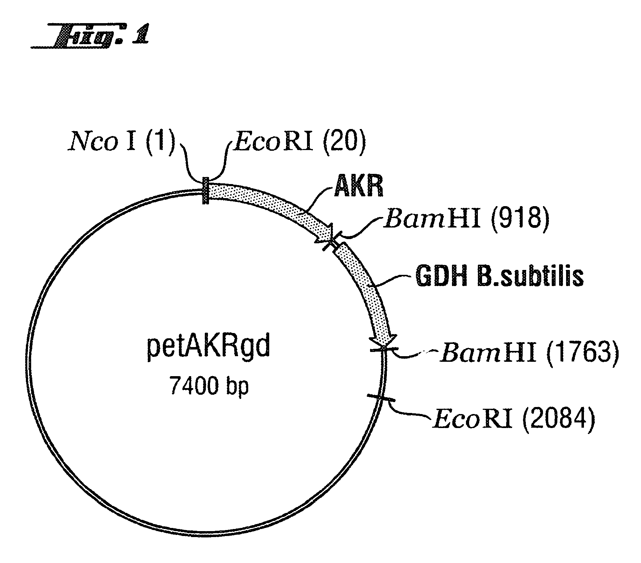

Das Enzym AKR aus der Bäckerhefe ist in der öffentlich zugänglichen GenBank Gendatenbank offenbart unter der Zugangsnummer X80642 (Genbezeichnung YPR1) und kann nach dem Stand der Technik aus genomischer DNS der Bäckerhefe isoliert werden. Die GDH-Mutante GDBS-E96A ist offenbart in DE 102004059376. Es wurde das in das Expressionsplasmid pET16B (Novagen) klonierte Tandemkonstrukt pAKRgd, bestehend aus dem AKR-Gen und gefolgt von einer Ribosomenbindestelle und daran anschließend die GDH-Mutante GDBS-E96A verwendet (Vektorkarte siehe Fig. 1). Bei der Klonierung wurde den Empfehlungen des Herstellers des Expressionsvektors pET16B gefolgt.

Fermentation von LB-ADH, T-ADH und AKR/GDH produzierenden E. coli:

### Herstellung eines Inokulums für die Fermentation:

1. Vorkultur von mit dem Plasmid pADH-1 (Enzym LB-ADH), bzw. pET24a[ADH-TS] (Enzym T-ADH) oder pAKRgd transformierten E. coli in LBamp Medium. Die Anzucht erfolgte für 7 bis 8 h auf einem Orbitalschüttler (Infors) bei 120 rpm und 30°C.

LBamp Medium enthielt Pepton vegetable (Oxoid) 10 g/l; Hefeextrakt (Oxoid) 5 g/l; NaCl 5 g/l und Ampicillin 0,1 g/l.

2. Vorkultur: 100 ml SM3amp Medium wurden in einem 1 1 Erlenmeyerkolben mit 1,3 ml Schüttelkultur beimpft. Die Anzucht erfolgte für 16 - 18 h bei 30°C und 120 rpm auf einem Orbitalschüttler bis zu einer Zelldichte OD₆₀₀/ml von 7 - 10. 100 ml der Vorkultur wurden zum Beimpfen von 1 1 Fermentermedium verwendet.

SM3amp Medium enthielt Pepton vegetable (Oxoid) 5 g/l; Hefeextrakt (Oxoid) 2,5 g/l; NaCl 0,1 g/l; Ammoniumsulfat 5 g/l; KH₂PO₄ 3 g/l; K₂HPO₄ 12 g/l; Glucose 5 g/l; MgSO₄ x 7 H₂O 0,3 g/l; CaCl₂ x 2 H₂O 14,7 mg/l; FeSO₄ x 7 H₂O 2 mg/l; Natriumcitrat x 2 H₂O 1 g/l; Vitamin B1 5 mg/l; Spurenelementemix 1 ml/l und Ampicillin 0,1 g/l.

Der Spurenelementemix hatte die Zusammensetzung H₃BO₃ 2,5 g/l; CoCl₂ x 6 H₂O 0,7 g/l; CuSO₄ x 5 H₂O 0,25 g/l; MnCl₂ x 4 H₂O 1,6 g/l; ZnSO₄ x 7 H₂O 0,3 g/l und Na₂MoO₄ x 2 H₂O 0,15 g/l.

Die Fermentationen wurden in Biostat CT Fermentern der Fa. Sartorius BBI Systems GmbH durchgeführt. Fermentationsmedium war FM2amp. Die Fermentation erfolgte im sog. Fed-Batch Modus.

FM2amp Medium enthielt Glucose 20 g/l; Pepton vegetable (Oxoid) 5 g/l; Hefeextrakt (Oxoid) 2,5 g/l; Ammoniumsulfat 5 g/l; NaCl 0,5 g/l; FeSO₄ x 7 H₂O 75 mg/l; Na₃Citrat x 2 H₂O 1 g/l; CaCl₂ x 2 H₂O 14,7 mg/l; MgSO₄ x 7 H₂O 0,3 g/l; KH₂PO₄ 1,5 g/l; Spurenelementemix 10 ml/l; Vitamin B1 5 mg/l und Ampicillin 0,1 g/l. Der pH des FM2amp Mediums wurde vor Beginn der Fermentation auf 7,0 eingestellt. Bei der Fermentation des T-ADH-Stammes enthielt das FM2amp Medium außerdem 2 mM ZnSO₄ x 7 H₂O.

1 l FM2amp wurde mit 100 ml Inokulum beimpft. Fermentationstemperatur war 30°C. pH der Fermentation war 7,0 und wurde mit den Korrekturmitteln 25 % NH₄OH, bzw. 6 N H₃PO₄ konstant gehalten. Die Belüftung erfolgte mit Pressluft bei einem konstanten Durchfluss von 5 slpm (Standard Liter pro Minute). Der Sauerstoffpartialdruck pO₂ wurde auf 50% Sättigung eingestellt. Die Regulierung des Sauerstoffpartialdruckes erfolgte über die Rührgeschwindigkeit (Rührerdrehzahl 450 - 1.300 rpm). Zur Kontrolle der Schaumbildung wurde Struktol J673 (20-25 % v/v in Wasser) verwendet.

Im Verlauf der Fermentation wurde der Glucoseverbrauch durch off-line Glucose-Messung mit einem Glucose-Analysator der Fa. YSI bestimmt. Sobald die Glukosekonzentration der Fermentationsansätze ca. 5 g/l betrug (5 - 6 h nach Inokulation), wurde die Zudosierung einer 60% w/w Glucose Feedlösung gestartet. Die Flußrate des Feeds wurde so gewählt, dass während der Produktionsphase eine Glukosekonzentration von 1 - 5 g/l eingehalten werden konnte.

Induktion der Produktion des LB-ADH bzw. T-ADH Enzyms oder der AKR/GDH Enzyme erfolgte durch Zugabe von IPTG (Stammlösung 100 mM) in einer Konzentration von 0,4 - 0,8 mM, sobald das Zellwachstum im Fermenter eine OD₆₀₀/ml von 50 - 60 erreicht hatte. Die gesamte Fermentationsdauer betrug 32 h. Nach Beendigung der Fermentation wurde die Fermenterbrühe (Trockenbiomasse 50 g/l) in Aliquots zu je 100 ml eingefroren.

Herstellung von Enzym-Rohextrakten aus Fermenterzellen: 2 ml Zellsuspension aus der Fermentation wurden zentrifugiert (10 min 3.000 rpm bei 4°C, Heraeus Fresco Zentrifuge). Das Sediment wurde in 1 ml 50 mM Kaliumphosphat, pH 7,0, 1 mM MgCl₂ resuspendiert und durch zwei Passagen durch einen "French Press" Homogenisator bei 800 bar Druck aufgeschlossen.

Das Homogenat wurde zentrifugiert (10 min 3.000 rpm bei 4°C, Heraeus Fresco Zentrifuge). Der Überstand ergab einen Enzym-Rohextrakt von 1 ml Volumen. Die Bestimmung der LB-ADH Aktivität ergab eine Volumenaktivität von 1.300 U/ml bzw. eine spezifische Aktivität von 108 U/mg Protein im Rohextrakt. Die Bestimmung der T-ADH Aktivität ergab eine Volumenaktivität von 36 U/ml bzw. eine spezifische Aktivität von 3 U/mg Protein im Rohextrakt. Die Bestimmung der AKR Aktivität ergab eine Volumenaktivität von 22 U/ml bzw. eine spezifische Aktivität von 1,8 U/mg Protein im Rohextrakt. Die Bestimmung der GDH Aktivität ergab eine Volumenaktivität von 71 U/ml bzw. eine spezifische Aktivität von 5,9 U/mg Protein im Rohextrakt.

Spektralphotometrische Bestimmung der LB-ADH und AKR Aktivität: Der Messansatz von 1 ml Volumen zur photometrischen Bestimmung von LB-ADH Aktivität war zusammengesetzt aus Messpuffer (0,1 M Kaliumphosphat, pH 7,0, 0,1 M NaCl, 1 mM MgCl₂), 3 µl Substrat 4-Cl-Acetessigsäureethylester, 0,2 mM NADPH und LB-ADH-haltigem Zellextrakt. Messtemperatur war 25°C. Die Reaktion wurde gestartet durch Zugabe des LB-ADH Zellextrakts und die Extinktionsabnahme infolge des Verbrauchs von NADPH bei einer Wellenlänge von 340 nm gemessen (Extinktionskoeffizient von NADPH: ε = 0,63 x 10⁴ l x Mol⁻¹ x cm⁻¹). Ein Unit LB-ADH bzw. AKR-Aktivität ist definiert als der Verbrauch von 1 µmol NADPH/min unter Testbedingungen.

Spektralphotometrische Bestimmung der T-ADH Aktivität: Der Messansatz von 1 ml Volumen zur photometrischen Bestimmung von T-ADH Aktivität war zusammengesetzt aus Messpuffer (0,1 M Kaliumphosphat, pH 7,0, 0,1 M NaCl, 1 mM MgCl₂), 3 µl Substrat Aceton, 0,2 mM NADPH und T-ADH-haltigem Zellextrakt. Messtemperatur war 25°C. Die Reaktion wurde gestartet durch Zugabe des T-ADH Zellextrakts und die Extinktionsabnahme infolge des Verbrauchs von NADPH bei einer Wellenlänge von 340 nm gemessen (Extinktionskoeffizient von NADPH: ε = 0,63 x 10⁴ l x Mol⁻¹ x cm⁻¹). Ein Unit T-ADH-Aktivität ist definiert als der Verbrauch von 1 µmol NADPH/min unter Testbedingungen.

GDH-Aktivität wurde bestimmt wie in DE102004059376 (Beispiel 3.) beschrieben.

Zur Bestimmung der spezifischen Aktivität wurde die Proteinkonzentration der Zellextrakte in an sich bekannter Weise mit dem sog. "BioRad Proteinassay" der Fa. BioRad bestimmt.

### 2. Beispiel:

### Herstellung von (R)-3-Hydroxy-Buttersäuremethylester aus Acetessigsäuremethylester durch Biotransformationen mit LB-ADH Zellen

1. Ansatz: Der Reaktionsansatz war zusammengesetzt aus 20 L (20.4 kg) Acetessigsäuremethylester (AcMe), 20 L (15,7 kg) Isopropanol, 1 kg Celite®, 3 L LB-ADH Zellen (Fermenterbrühe wie im 1. Beispiel beschrieben), 50 µM NADP und 7 L KPi-Puffer. Die Zusammensetzung von KPi-Puffer war 0,1 M Kaliumphosphat, pH 7,0, 0,1 M NaCl, 1 mM MgCl₂. Der Reaktionsansatz wurde in einem 100 L Reaktionskessel bei 30°C gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz durch Druckfiltration über eine Stahlnutsche der Fa. Seitz filtriert. Im Filtrat betrug der Reaktionsumsatz des eingesetzten AcMe 94,4 %. Der Enantiomerenüberschuss ee des Produktes (R)-3-Hydroxy-Buttersäurmethylester betrug 100 %. Der Celite®-Enzym-Filterkuchen wurde für den 2. Ansatz in den Reaktionskessel zurückgeführt.

Die Reaktionsanalyse durch chirale GC wurde durchgeführt, wobei ein Gaschromatograph 6890N der Fa. Agilent verwendet wurde, ausgerüstet mit einer Chiraldex^{™} G-TA der Fa. Astec (20 m x 0,32 mm) zur chiralen Trennung.

Zur gaschromatochraphischen Trennung wurde ein Temperaturgradient von 65°C - 170°C mit einer Gradientensteilheit von 10°C/min eingestellt. Retentionszeiten unter diesen Bedingungen waren:
(S)-3-Hydroxy-Buttersäuremethylester: 5,9 min.
(R)-3-Hydroxy-Buttersäuremethylester: 6,5 min.
Acetessigsäuremethylester: 11,6 min.

2. Ansatz: Der Reaktionsansatz war zusammengesetzt aus 20 L (20.4 kg) Acetessigsäuremethylester (AcMe), 20 L (15,7 kg) Isopropanol, dem aus dem 1. Ansatz zurückgewonnenen Celite®-Enzym-Filterkuchen, 450 ml LB-ADH Zellen (Fermenterbrühe wie im 1. Beispiel beschrieben), 50 µM NADP und 9,8 L KPi-Puffer. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz filtriert (siehe 1. Ansatz). Im Filtrat betrug der Reaktionsumsatz des eingesetzten AcMe 94,3 %. Der Enantiomerenüberschuss ee des Produktes (R)-3-Hydroxy-Buttersäuremethylester betrug 100 %. Der Celite®-Enzymfilterkuchen wurde für den 3. Ansatz in den Reaktionskessel zurückgeführt.

3. Ansatz: Der Reaktionsansatz war zusammengesetzt aus 20 L (20.4 kg) Acetessigsäuremethylester (AcMe), 20 L (15,7 kg) Isopropanol, dem aus dem 2. Ansatz zurückgewonnenen Celite®-Enzym-Filterkuchen, 450 ml LB-ADH Zellen (Fermenterbrühe wie im 1. Beispiel beschrieben), 50 µM NADP und 9,8 L KPi-Puffer. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz filtriert (siehe 1. Ansatz). Im Filtrat betrug der Reaktionsumsatz des eingesetzten AcMe 94,6 %. Der Enantiomerenüberschuss ee des Produktes (R)-3-Hydroxy-Buttersäuremethylester betrug 100 %. Der Celite®-Enzymfilterkuchen wurde für den 4. Ansatz in den Reaktionskessel zurückgeführt.

4. Ansatz: Der Reaktionsansatz war zusammengesetzt aus 20 L (20.4 kg) Acetessigsäuremethylester (AcMe), 20 L (15,7 kg) Isopropanol, dem aus dem 3. Ansatz zurückgewonnenen Celite®-Enzym-Filterkuchen, 450 ml LB-ADH Zellen (Fermenterbrühe wie im 1. Beispiel beschrieben), 50 µM NADP und 9,8 L KPi-Puffer. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz filtriert (siehe 1. Ansatz). Im Filtrat betrug der Reaktionsumsatz des eingesetzten AcMe 94,5 %. Der Enantiomerenüberschuss ee des Produktes (R)-3-Hydroxy-Buttersäuremethylester betrug 100 %.

Die aus den vier Ansätzen gewonnenen Filtrate wurden vereinigt (179,7 kg) und das Produkt (R)-3-Hydroxy-Buttersäuremethylester durch Destillation gewonnen. Dabei wurden in einer Siebboden Destillationsapparatur zuerst leichtflüchtige Bestandteile abdestilliert (Temperatur bis zu 100°C, Vakuum bis zu 150 mbar) gefolgt von einer Feindestillation (Temperatur bis zu 120°C, Vakuum bis zu 120 mbar). Die Ausbeute an (R)-3-Hydroxy-Buttersäuremethylester betrug 63 kg (77,2 % Ausbeute bezogen auf insgesamt 81,6 kg eingesetztes AcMe).

### 3. Beispiel

### Herstellung von (R)-1-Acetoxy-2-Propanol aus Acetoxyaceton durch Biotransformation mit LB-ADH Zellen

1. Ansatz: Der Reaktionsansatz war zusammengesetzt aus 20 L (41,5 kg) Acetoxyaceton, 20 L (15,7 kg) Isopropanol, 1 kg Celite®, 10 L LB-ADH Zellen, 50 µM NADP und 1 L KPi-Puffer. Die Zusammensetzung von KPi-Puffer war 0,1 M Kaliumphosphat, pH 7,0, 0,1 M NaCl, 1 mM MgCl₂. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz filtriert (siehe 2. Beispiel). Im Filtrat betrug der Reaktionsumsatz des eingesetzten Acetoxyacetons 93,9 %. Der Enantiomerenüberschuss ee des Produktes (R)-1-Acetoxy-2-Propanol betrug 100 %.

Die Reaktionsanalyse durch chirale GC wurde durchgeführt, wie in Co10503 offenbart, wobei ein Gaschromatograph 6890N der Fa. Agilent verwendet wurde, ausgerüstet mit einer CP-Chirasil-Dex-CB Säule der Fa. Varian (25 m x 0,25 mm) zur chiralen Trennung.

Zur gaschromatochraphischen Trennung wurde ein Temperaturgradient von 100°C - 140°C mit einer Gradientensteilheit von 2°C/min, gefolgt von einem Temperaturgradienten von 140°C - 170°C mit einer Gradientensteilheit von 10°C/min eingestellt. Retentionszeiten unter diesen Bedingungen waren:
Acetoxyaceton: 4,4 min.
(R)-1-Acetoxy-2-Propanol: 6,2 min.
(S)-1-Acetoxy-2-Propanol: 6,4 min.
(R)-2-Acetoxy-1-Propanol: 7,8 min.
(S)-2-Acetoxy-1-Propanol: 8,7 min.

2. Ansatz: Der Reaktionsansatz war zusammengesetzt aus 20 L (41,5 kg) Acetoxyaceton, 20 L (15,7 kg) Isopropanol, dem aus dem 1. Ansatz zurückgewonnenen Celite®-Enzym-Filterkuchen, 1,5 L LB-ADH Zellen (Fermenterbrühe wie im 1. Beispiel beschrieben), 50 µM NADP und 8 L KPi-Puffer. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz filtriert (siehe 2. Beispiel). Im Filtrat betrug der Reaktionsumsatz des eingesetzten Acetoxyacetons 95,0 %. Der Enantiomerenüberschuss ee des Produktes (R)-1-Acetoxy-2-Propanol betrug 100 %. Der Celite®-Enzymfilterkuchen wurde für den 3. Ansatz in den Reaktionskessel zurückgeführt.

3. Ansatz: Der Reaktionsansatz war zusammengesetzt aus 20 L (41,5 kg) Acetoxyaceton, 20 L (15,7 kg) Isopropanol, dem aus dem 2. Ansatz zurückgewonnenen Celite®-Enzym-Filterkuchen, 1,5 L LB-ADH Zellen (Fermenterbrühe wie im 1. Beispiel beschrieben), 50 µM NADP und 8 L KPi-Puffer. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz filtriert (siehe 2. Beispiel). Im Filtrat betrug der Reaktionsumsatz des eingesetzten Acetoxyacetons 97,1 %. Der Enantiomerenüberschuss ee des Produktes (R)-1-Acetoxy-2-Propanol betrug 100 %. Der Celite®-Enzymfilterkuchen wurde für den 4. Ansatz in den Reaktionskessel zurückgeführt.

4. Ansatz: Der Reaktionsansatz war zusammengesetzt aus 20 L (41,5 kg) Acetoxyaceton, 20 L (15,7 kg) Isopropanol, dem aus dem 3. Ansatz zurückgewonnenen Celite®-Enzym-Filterkuchen, 1,5 L LB-ADH Zellen (Fermenterbrühe wie im 1. Beispiel beschrieben), 50 µM NADP und 8 L KPi-Puffer. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz filtriert (siehe 2. Beispiel). Im Filtrat betrug der Reaktionsumsatz des eingesetzten Acetoxyacetons 97,5 %. Der Enantiomerenüberschuss ee des Produktes (R)-1-Acetoxy-2-Propanol betrug 100 %.

Die aus den vier Ansätzen gewonnenen Filtrate wurden vereinigt (183,8 kg) und im Vakuum destilliert, um leichtflüchtige Reaktionsprodukte (Isopropanol, Aceton) und restliches Wasser aus dem Rohprodukt zu entfernen.

Die Ausbeute an (R)-1-Acetoxypropanol betrug 74,4 kg (86,5 % Ausbeute bezogen auf insgesamt 86 kg eingesetztes Acetoxyaceton).

### 4. Beispiel:

### Herstellung von (S)-3-Hydroxy-Buttersäuremethylester aus Acetessigsäuremethylester durch Biotransformationen mit T-ADH Zellen

1. Ansatz: Der Reaktionsansatz war zusammengesetzt aus 40 ml (40.8 g) Acetessigsäuremethylester (AcMe), 40 ml (31,4 g) Isopropanol, 2 g Celite®, 6 ml T-ADH Zellen (Fermenterbrühe wie im 1. Beispiel beschrieben), 50 µM NADP, 2 ml Glycerin und 12 ml KPi-Puffer. Die Zusammensetzung von KPi-Puffer war 0,1 M Kaliumphosphat, pH 7,0, 0,1 M NaCl, 1 mM MgCl₂. Der Reaktionsansatz wurde in einem 100 ml Reaktionsgefäß bei 30°C gerührt. Zu verschiedenen Zeitpunkten wurden 0,1 ml Proben des Reaktionsansatzes genommen, mit 1 ml MTBE extrahiert und durch chirale GC analysiert (siehe 2. Beispiel). Nach 24 h wurde der Ansatz filtriert (Vakuumfiltration über eine Porzellanfritte). Im Filtrat betrug der Reaktionsumsatz des eingesetzten AcMe 92,1 %. Der Enantiomerenüberschuss ee des Produktes (S)-3-Hydroxy-Buttersäuremethylester betrug 100 %. Der Celite®-Enzym-Filterkuchen wurde für den 2. Ansatz in das Reaktionsgefäss zurückgeführt.

2. Ansatz: Der Reaktionsansatz war zusammengesetzt aus 40 ml (40.8 g) Acetessigsäuremethylester (AcMe), 40 ml (31,4 g) Isopropanol, dem aus dem 1. Ansatz zurückgewonnenen Celite®-Enzym-Filterkuchen, 1,5 ml T-ADH Zellen (Fermenterbrühe wie im 1. Beispiel beschrieben), 50 µM NADP, 2 ml Glycerin und 16 ml KPi-Puffer. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz filtriert (siehe 1. Ansatz). Im Filtrat betrug der Reaktionsumsatz des eingesetzten AcMe 92,2 %. Der Enantiomerenüberschuss ee des Produktes (S)-3-Hydroxy-Buttersäuremethylester betrug 100 %. Der Celite®-Enzym-Filterkuchen wurde für den 3. Ansatz in das Reaktionsgefäß zurückgeführt.

3. Ansatz: Der Reaktionsansatz war zusammengesetzt aus 40 ml (40.8 g) Acetessigsäuremethylester (AcMe), 40 ml (31,4 g) Isopropanol, dem aus dem 2. Ansatz zurückgewonnenen Celite®-Enzym-Filterkuchen, 1,5 ml T-ADH Zellen (Fermenterbrühe wie im 1. Beispiel beschrieben), 50 µM NADP, 2 ml Glycerin und 16 ml KPi-Puffer. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz filtriert (siehe 1. Ansatz). Im Filtrat betrug der Reaktionsumsatz des eingesetzten AcMe 92,6 %. Der Enantiomerenüberschuss ee des Produktes (S)-3-Hydroxy-Buttersäuremethylester betrug 100 %.

4. Ansatz: Der Reaktionsansatz war zusammengesetzt aus 40 ml (40.8 g) Acetessigsäuremethylester (AcMe), 40 ml (31,4 g) Isopropanol, dem aus dem 3. Ansatz zurückgewonnenen Celite®-Enzym-Filterkuchen, 1,5 ml T-ADH Zellen (Fermenterbrühe wie im 1. Beispiel beschrieben), 50 µM NADP, 2 ml Glycerin und 16 ml KPi-Puffer. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz filtriert (siehe 1. Ansatz). Im Filtrat betrug der Reaktionsumsatz des eingesetzten AcMe 92,8 %. Der Enantiomerenüberschuss ee des Produktes (S)-3-Hydroxy-Buttersäuremethylester betrug 100 %.

### 5. Beispiel:

### Herstellung von (S)-4-Chlor-3-Hydroxy-Buttersäureethylester aus 4-Cl-Acetessigsäureethylester durch Biotransformation mit LB-ADH Zellen

Ansatz ohne Celite®: Der Reaktionsansatz war zusammengesetzt aus 20 ml (24,3 g) 4Cl-Acetessigsäureethylester (4Cl-ACE), 20 ml n-Butylacetat, 40 ml (31,4 g) Isopropanol, 4 ml LB-ADH Zellen (Fermenterbrühe wie im 1. Beispiel beschrieben), 50 µM NADP, und 16 ml KPiC-Puffer. Die Zusammensetzung von KPiC-Puffer war 0,1 M Kaliumphosphat, pH 7,0, 0,1 M NaHCO₃, 1 mM MgCl₂. Der Reaktionsansatz wurde in einem 100 ml Reaktionsgefäss bei Raumtemperatur gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz filtriert (Vakuumfiltration über eine Fritte). Im Filtrat betrug der Reaktionsumsatz des eingesetzten 4Cl-ACE 57,3 %. Der Enantiomerenüberschuss ee des Produktes (S)-4-Chlor-3-Hydroxy-Buttersäureethylester (S-CHBE) betrug 100 %. Der zeitliche Reaktionsverlauf ist in Tab. 1 dargestellt.

Zur Reaktionsanalyse durch chirale GC wurde ein Gaschromatograph 6890N der Fa. Agilent verwendet, ausgerüstet mit einer Chiraldex^{™} G-TA Säule der Fa. Astec (20 m x 0,32 mm) zur chiralen Trennung.

Zur gaschromatochraphischen Trennung wurde ein Temperaturgradient von 105°C - 150°C mit einer Gradientensteilheit von 10°C/min eingestellt. Retentionszeiten unter diesen Bedingungen waren:
4-Cl-Acetessigsäureethylester: 4,5 min.
(R)-4-Cl-3-Hydroxy-Buttersäureethylester: 5,2 min.
(S)-4-Cl-3-Hydroxy-Buttersäureethylester: 5,7 min.

Ansatz A mit Celite®: Der Reaktionsansatz war zusammengesetzt aus 20 ml (24,3 g) 4Cl-ACE, 20 ml n-Butylacetat, 40 ml (31,4 g) Isopropanol, 2 g Celite®, 4 ml LB-ADH Zellen (Fermenterbrühe wie im 1. Beispiel beschrieben), 50 µM NADP, und 16 ml KPiC-Puffer. Der Reaktionsansatz wurde in einem 100 ml Reaktionsgefäss bei Raumtemperatur gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz filtriert (Vakuumfiltration über eine Fritte). Im Filtrat betrug der Reaktionsumsatz des eingesetzten 4Cl-ACE 100 %. Der Enantiomerenüberschuss ee des Produktes S-CHBE betrug 100 %. Der zeitliche Reaktionsverlauf ist in Tab. 1 dargestellt.

Ein Vergleich der in Tab. 1 dargestellten Reaktionsverläufe zeigt, dass das LB-ADH Enzym durch den Celite®-Zusatz stabilisiert wird und dadurch eine Biotransformation mit deutlich höherer Raum-Zeitausbeute ermöglicht wird.

**Tab. 1**

| | | | |
|---|---|---|---|
| Reaktionsverlauf der S-CHBE Synthese mit LB-ADH mit und ohne Zusatz von Celite® | | | |

| Ansatz ohne Celite® | | Ansatz mit Celite® | |
|---|---|---|---|
| Zeit (h) | Umsatz 4Cl-ACE zu S-CHBE | Zeit (h) | Umsatz 4Cl-ACE zu S-CHBE |
| 0 | 0,4 | 0 | 0,9 |
| 2 | 10,8 | 2 | 40 |
| 5 | 31,6 | 4 | 69,8 |
| 7 | 42,2 | 21 | 99,9 |
| 24 | 57,3 | 23 | 99,9 |

Wiedereinsatz des Celite®-LB-ADH Filterkuchens: Der Ansatz mit Celite® wurde dreimal wiederholt, wobei jeweils der Celite®-LB-ADH Filterkuchen aus dem vorhergehenden Ansatz wiederverwendet wurde und zusätzlich jeweils 2 ml frische Fermenterzellen zudosiert wurden. Im Filtrat betrug der Reaktionsumsatz des eingesetzten 4C1-ACE jeweils 100 %. Der Enantiomerenüberschuss ee des Produktes S-CHBE betrug 100 %.

Ansatz B mit Celite®: Der Reaktionsansatz war zusammengesetzt aus 40 ml (48,6 g) 4Cl-ACE, 15 ml n-Butylacetat, 25 ml (19,6 g) Isopropanol, 2 g Celite®, 10 ml LB-ADH Zellen (Fermenterbrühe wie im 1. Beispiel beschrieben), 50 µM NADP, und 10 ml KPiC-Puffer. Der Reaktionsansatz wurde in einem 100 ml Reaktionsgefäss bei Raumtemperatur gerührt. Zu verschiedenen Zeitpunkten wurden 0,1 ml Proben des Reaktionsansatzes genommen, mit 1 ml MTBE extrahiert und durch chirale GC analysiert. Nach 24 h wurde der Ansatz filtriert (Vakuumfiltration über eine Fritte). Im Filtrat betrug der Reaktionsumsatz des eingesetzten 4Cl-ACE 100 %. Der Enantiomerenüberschuss ee des Produktes S-CHBE betrug 100 %.

### 6. Beispiel:

### Herstellung von (S)-2-Hexanol aus 2-Hexanon durch Biotransformtion mit T-ADH Zellen

1. Ansatz: Der Reaktionsansatz war zusammengesetzt aus 15 ml (12,2 g) 2-Hexanon, 50 ml (39,3 g) Isopropanol, 2 ml T-ADH Zellen (Fermenterbrühe wie im 1. Beispiel beschrieben), 50 µM NADP, 2 g Celite® und 33 ml KPi-Puffer. Die Zusammensetzung von KPI-Puffer war 0,1 M Kaliumphosphat, pH 7,0, 0,1 M NaCl, 1 mM MgCl₂. Der Reaktionsansatz wurde in einem 100 ml Reaktionsgefäss bei 30°C gerührt.

Zu verschiedenen Zeitpunkten wurden 0,1 ml Proben des Reaktionsansatzes genommen, mit 1 ml Essigester extrahiert und durch chirale GC analysiert. Nach 24 h betrug der Reaktionsumsatz des eingesetzten 2-Hexanon 80 %. Der Enantiomerenüberschuss ee des Produktes (S)-2-Hexanol betrug 100 %.

Zur Reaktionsanalyse durch chirale GC wurde ein Gaschromatograph 6890N der Fa. Agilent verwendet, ausgerüstet mit einer Chiraldex^{™} G-TA Säule der Fa. Astec (10 m x 0,32 mm) zur chiralen Trennung.

Zur gaschromatographischen Trennung wurde ein Temperaturgradient von 50°C - 70°C mit einer Gradientensteilheit von 5°C/min, gefolgt von 70°C - 80°C mit einer Gradientensteilheit von 20°C/min eingestellt. Retentionszeiten unter diesen Bedingungen waren:
2-Hexanon: 4,30 min.
(R)-2-Hexanol: 1,88 min.
(S)-2-Hexanol: 2,22 min.

Wiedereinsatz des Celite®-T-ADH Filterkuchens: Der 1. Ansatz wurde dreimal wiederholt, wobei jeweils der Celite®-T-ADH Filterkuchen aus dem vorhergehenden Ansatz wiederverwendet wurde und zusätzlich jeweils 0,3 ml frische Fermenterzellen zudosiert wurden. Im Filtrat betrug der Reaktionsumsatz des eingesetzten 2-Hexanon zwischen 79 - 83 %. Der Enantiomerenüberschuss ee des Produktes (S)-2-Hexanol betrug jeweils 100 %.

### 7. Beispiel:

### Herstellung von (S)-3-Hydroxybutyraldehyd-Dimethylacetal (S-HBDMA) aus Acetyl-Acetaldeyddimethylacetal (AADMA) durch Biotransformation mit AKRgd Zellen

1. Ansatz: Der Reaktionsansatz war zusammengesetzt aus 5 ml (5 g) Acetyl-Acetaldeyddimethylacetal (AADMA), 8 ml AKRgd Zellen (Fermenterbrühe wie im 1. Beispiel beschrieben), 50 µM NADP, 2 g Celite® und 85 ml KpiG-Puffer. Die Zusammensetzung von KpiG-Puffer war 0,1 M Kaliumphosphat, pH 7,0, 0,1 M NaCl, 1 M Glucose. Der Reaktionsansatz wurde in einem 100 ml Reaktionsgefäß bei 30°C gerührt. Der pH von 7,0 wurde konstant gehalten durch einen Titrator ("TitroLine alpha" der Fa. Schott), über den 10 M KOH zudosiert wurde.

Zu verschiedenen Zeitpunkten wurden 0,1 ml Proben des Reaktionsansatzes entnommen, mit 1 ml MTBE extrahiert und durch chirale GC analysiert. Nach 24 h betrug der Reaktionsumsatz des eingesetzten AADMA 93,4 %. Der Enantiomerenüberschuss ee des Produktes S-HBDMA betrug 100 %.

Zur Reaktionsanalyse durch chirale GC wurde ein Gaschromatograph 6890N der Fa. Agilent verwendet, ausgerüstet mit einer CP-Chirasil-Dex-CB Säule der Fa. Varian (25 m x 0,25 mm) zur chiralen Trennung.

Zur gaschromatographischen Trennung wurde ein Temperaturgradient von 100°C - 140°C mit einer Gradientensteilheit von 2°C/min, gefolgt von 140°C - 170°C mit einer Gradientensteilheit von 10°C/min eingestellt. Retentionszeiten unter diesen Bedingungen waren:
Acetyl-Acetaldehyddimethylacetal: 5,18 min.
(S)-3-Hydroxy-Butyralddehyd-Dimethylacetal: 7,42 min.
(R)-3-Hydroxy-Butyralddehyd-Dimethylacetal: 7,66 min.

Wiedereinsatz des Celite®-AKRgd Filterkuchens: Der 1. Ansatz wurde dreimal wiederholt, wobei jeweils der Celite®-AKRgd Filterkuchen aus dem vorhergehenden Ansatz wiederverwendet wurde und zusätzlich jeweils 2 ml frische Fermenterzellen zudosiert wurden. Im Filtrat betrug der Reaktionsumsatz des eingesetzten AADMA zwischen 93 - 95 %. Der Enantiomerenüberschuss ee des Produktes S-HBDMA betrug jeweils 100 %.

### 8. Beispiel (Vergleichsbeispiel):

### Wiedereinsatz von LB-ADH nach Extraktion des Reaktionsansatzes bei der Herstellung von (R)-3-Hydroxy-Buttersäuremethylester aus Acetessigsäuremethylester

1. Ansatz: Der Reaktionsansatz war zusammengesetzt aus 40 ml (40.8 g) Acetessigsäuremethylester (AcMe), 40 ml (31,4 g) Isopropanol, 6 ml LB-ADH Zellen (Fermenterbrühe wie im 1. Beispiel beschrieben), 50 µM NADP und 14 ml KPi-Puffer. Die Zusammensetzung von KPi-Puffer war 0,1 M Kaliumphosphat, pH 7,0, 0,1 M NaCl, 1 mM MgCl₂. Die Zusammensetzung war equivalent zu der Biotransformation aus dem 2. Beispiel (1. Ansatz), jedoch ohne Zusatz von Celite®. Der Reaktionsansatz wurde in einem 100 ml Reaktionsgefäß bei 30°C gerührt. Zu verschiedenen Zeitpunkten wurden 0,1 ml Proben des Reaktionsansatzes genommen, mit 1 ml MTBE extrahiert und durch chirale GC analysiert (siehe 2. Beispiel). Nach 24 h wurde der Ansatz beendet. Der Reaktionsumsatz des eingesetzten AcMe betrug 96,2 %. Der Enantiomerenüberschuss ee des Produktes (R)-3-Hydroxy-Buttersäurmethylester betrug 100 %.

Der Reaktionsansatz wurde dreimal mit je 100 ml MTBE extrahiert und die enzymhaltige wässrige Phase wiedergewonnen. Das Volumen der wässrigen Phase betrug 14 ml.

2. Ansatz: Der Reaktionsansatz war zusammengesetzt aus 40 ml (40.8 g) Acetessigsäuremethylester (AcMe), 40 ml (31,4 g) Isopropanol, 14 ml LB-ADH-haltige, wässrige Phase nach der MTBE-Extraktion des 1. Ansatzes, 0,9 ml LB-ADH Zellen (Fermenterbrühe wie im 1. Beispiel beschrieben, ist equivalent zu der Menge Zellen, mit der im 2. Beispiel der 2. Ansatz aufgestockt wurde), 50 µM NADP und 5,1 ml KPi-Puffer. Der Reaktionsansatz wurde in einem 100 ml Reaktionsgefäß bei 30°C gerührt. Zu verschiedenen Zeitpunkten wurden 0,1 ml Proben des Reaktionsansatzes genommen, mit 1 ml MTBE extrahiert und durch chirale GC analysiert (siehe 2. Beispiel). Nach 24 h wurde der Ansatz beendet. Der Reaktionsumsatz des eingesetzten AcMe betrug 50,9 %. Der Enantiomerenüberschuss ee des Produktes (R)-3-Hydroxy-Buttersäurmethylester betrug 100 %.

Dieses Vergleichsbeispiel zeigt, dass der Wiedereinsatz des Enzyms nach Produktextraktion mit MTBE nur zu einer unvollständigen Reaktion führt. Wie im 2. Beispiel gezeigt, wird im Gegensatz dazu das Enzym durch Adsorption an Celite® unter ansonsten gleichen Bedingungen so stabilisiert, dass beim Wiedereinsatz ein nahezu vollständiger Reaktionsumsatz erzielt wird und das Enzym für noch weitere Reaktionszyklen zur Verfügung steht.

## Patentansprüche

1. Verfahren zur Herstellung eines chiralen sekundären Alkohols bei dem eine Biotransformationszusammensetzung, enthaltend ein Keton der Formel (I), 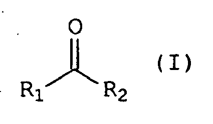 wobei R₁ und R₂ verschieden sind und jeweils organischer Rest bedeuten, eine Oxidoreduktase und ein Cosubstrat, zur Reaktion gebracht wird, wobei ein chiraler sekundärer Alkohol entsteht, **dadurch gekennzeichnet, dass** die Biotransformationszusammensetzung ein Adsorbens ausgewählt aus der Gruppe Aluminiumoxid, Aluminiumoxidhydrat, Siliziumdioxid, Kieselsäure, gefällte Kieselsäure, pyrogene Kieselsäure, Silikat, Calciumphosphat, kationische und anionische Ionenaustauscher auf Basis eines Polystyrolharzes, Polysaccharid, vernetztes Dextran, vernetzte Agarose Aktivkohle und Methacrylat enthält, welches mit der Oxidoreduktase assoziiert und welches nach dem Ende der Reaktion von der Biotransformationszusammensetzung abgetrennt wird und die mit dem Adsorbens assoziierte Oxidoreduktase für den Wiedereinsatz vom Reaktionsgemisch durch Filtration, Sedimentation oder Zentrifugation abgetrennt wird.

2. Verfahren gemäß Anspruch 1, **dadurch gekennzeichnet, dass** die Adsorbentien ausgewählt sind aus der Gruppe Aluminiumoxid, Kieselgel, Mg-Silicat, Bentonit, Celite^{®}, XAD, Dowex^{®}, Amberlite^{®}, Sepharose^{®}, Sephadex^{®}, Superose^{®}, Cellulose.

3. Verfahren gemäß Anspruch 2, **dadurch gekennzeichnet, dass** die Adsorbentien XAD, Florisil^{®}, Kieselgel, oder Celite^{®} sind.

4. Verfahren gemäß Anspruch 3, **dadurch gekennzeichnet, dass** das Adsorbens Celite^{®} ist.

5. Verfahren gemäß einem der Ansprüche 1 bis 4, **dadurch gekennzeichnet, dass** der chirale sekundäre Alkohol eine Verbindung der Formel (II) oder (III) 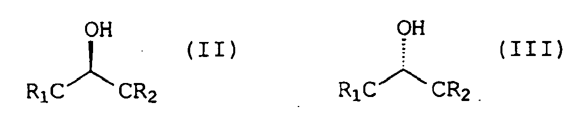 ist, wobei R₁ und R₂ voneinander verschieden sind und organischer Rest bedeuten.

6. Verfahren gemäß Anspruch 5, **dadurch gekennzeichnet, dass** R₁ und R₂ verschiedene organische Reste mit 1-20 C-Atomen, wobei ein oder mehrere C-Atome der Reste R₁ oder R₂ durch Atome, ausgewählt aus der Gruppe B, N, O, Si, P und S, ersetzt sein können oder durch F, Cl, Br, J, durch ggf. substituierte C₃-C₈-Cycloalkyl, C₆-C₂₀-Aryl, C₅-C₂₀-Heteroaryl, durch Silylreste sowie durch CN, NH₂, NO oder NO₂ substituiert sein können.

7. Verfahren gemäß einem der Ansprüche 1 bis 6, **dadurch gekennzeichnet, dass** die Oxidoreduktase eine Carbonylreduktase mit S- oder R-Spezifität ist.

8. Verfahren gemäß einem der Ansprüche 1 bis 7, **dadurch gekennzeichnet, dass** die Biotransformationszusammensetzung einen Redox-Cofaktor ausgewählt aus Verbindungen der Gruppe NAD, NADP, NADH, NADPH und deren Salzen umfasst.

9. Verfahren gemäß einem der Ansprüche 7 oder 8, **dadurch gekennzeichnet, dass** die Carbonylreduktase eine Alkohldehydrogenase ist und das Cosubstrat ein Alkohol ist.

10. Verfahren gemäß Anspruch 9, **dadurch gekennzeichnet, dass** der Alkohol Isopropanol oder 2-Butanol ist.

11. Verfahren gemäß Anspruch 1 bis 10, **dadurch gekennzeichnet, dass** Edukte der allgemeinen Formel (I) zu > 80 % zu einem chiralen sekundären Alkohol umgesetzt werden.

12. Verfahren gemäß Anspruch 1 bis 11, **dadurch gekennzeichnet, dass** das abgetrennte Adsorbens welches mit der Carbonylreduktase assoziiert ist, in eine Biotransformationszusammensetzung gemäß Anspruch 1 gegeben wird wobei der Biotransformationszusammensetzung keine Carbonylreduktase zugesetzt wird oder nur soviel Carbonylreduktase zugesetzt wird, wie bei der Abtrennung des Adsorbens verloren wurde und dieser zweite Reaktionszyklus analog Anspruch 1 durchgeführt wird.

13. Verfahren gemäß Anspruch 12, **dadurch gekennzeichnet, dass** bis zu 20 Reaktionszyklen unter Einsatz des im vorherigen Reaktioszyklus abgetrennten Adsorbens durchgeführt werden.

14. Verfahren gemäß Anspruch 1 bis 13, **dadurch gekennzeichnet, dass** der chirale sekundäre Alkohol aus dem Reaktionsansatz nach Abtrennung des Adsorbens durch Destillation oder mittels eines mit Wasser nicht mischbaren, organischen Lösungsmittels extrahiert wird.

## Claims

1. Method of producing a chiral secondary alcohol in which a biotransformation composition containing a ketone of the formula (I), 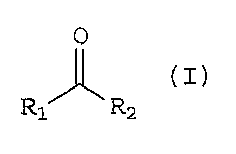 R₁ and R₂ being different and each being an organic radical, an oxidoreductase and a cosubstrate is reacted, a chiral secondary alcohol being formed, **characterized in that** the biotransformation composition contains an adsorbent selected from the group aluminium oxide, aluminium oxide hydrate, silicon dioxide, silicic acid, precipitated silicic acid, pyrogenic silica, silicate, calcium phosphate, cationic and anionic ion exchangers based on a polystyrene resin, polysaccharide, crosslinked dextran, crosslinked agarose, activated carbon and methacrylate, which adsorbent associates with the oxidoreductase and which is separated off from the biotransformation composition after the end of the reaction and the oxidoreductase associated with the adsorbent is separated off by filtration, sedimentation or centrifugation for reuse of the reaction mixture.

2. Method according to Claim 1, **characterized in that** the adsorbents are selected from the group aluminium oxide, silica gel, Mg silicate, bentonite, Celite^{®}, XAD, Dower^{®}, Amberlite^{®}, Sepharose^{®}, Sephadex^{®}, Superose^{®}, cellulose.

3. Method according to Claim 2, **characterized in that** the adsorbents are XAD, Florisil^{®}, silica gel, or Celite^{®}.

4. Method according to Claim 3, **characterized in that** the adsorbent is Celite^{®}.

5. Method according to one of Claims 1 to 4, **characterized in that** the chiral secondary alcohol is a compound of the formula (II) or (III) 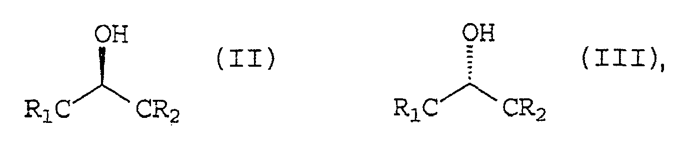 R₁ and R₂ being different from one another and being organic radicals.

6. Method according to Claim 5, **characterized in that** R₁ and R₂ are different organic radicals having 1-20 carbon atoms, one or more carbon atoms of the radicals R₁ or R₂ being able to be replaced by atoms selected from the group B, N, O, Si, P and S, or being able to be substituted by F, Cl, Br, I, by optionally substituted C₃-C₈-cycloalkyl, C₆-C₂₀-aryl, C₅-C₂₀-heteroaryl, by silyl radicals and also by CN, NH₂, NO or NO₂.

7. Method according to one of Claims 1 to 6, **characterized in that** the oxidoreductase is a carbonyl reductase having S or R specificity.

8. Method according to one of Claims 1 to 7, **characterized in that** the biotransformation composition comprises a redox cofactor selected from compounds of the group NAD, NADP, NADH, NADPH and salts thereof.

9. Method according to one of Claims 7 and 8, **characterized in that** the carbonyl reductase is an alcohol dehydrogenase and the cosubstrate is an alcohol.

10. Method according to Claim 9, **characterized in that** the alcohol is isopropanol or 2-butanol.

11. Method according to Claims 1 to 10, **characterized in that** starting materials of the general formula (I) are > 80% reacted to a chiral secondary alcohol.

12. Method according to Claims 1 to 11, **characterized in that** the adsorbent which has been separated off and is associated with the carbonyl reductase is placed into a biotransformation composition according to Claim 1, no carbonyl reductase being added to the biotransformation composition, or only as much carbonyl reductase being added as was lost in the separation of the adsorbent and this second reaction cycle being carried out in a similar manner to Claim 1.

13. Method according to Claim 12, **characterized in that** up to 20 reaction cycles are carried out using the adsorbent separated off in the previous reaction cycle.

14. Method according to Claims 1 to 13, **characterized in that** the chiral secondary alcohol from the reaction batch after separating off the absorbent is extracted by distillation or by means of a water-immiscible organic solvent.

## Revendications

1. Procédé pour la préparation d'un alcool secondaire chiral, dans lequel on fait réagir une composition de biotransformation, contenant une cétone de formule (I), 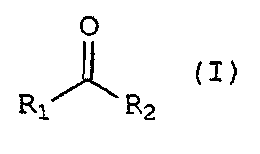 dans laquelle R₁ et R₂ sont différents et représentent chacun un radical organique, une oxydoréductase et un co-substrat, de sorte qu'il en résulte un alcool secondaire chiral, **caractérisé en ce que** la composition de biotransformation contient un adsorbant choisi dans le groupe constitué par l'oxyde d'aluminium, l'oxyde d'aluminium hydraté, le dioxyde de silicium, l'acide silicique, l'acide silicique précipité, l'acide silicique pyrogéné, un silicate, le phosphate de calcium, des échangeurs d'ions cationiques et échangeurs d'ions anioniques à base d'une résine polystyrène, un polysaccharide, le dextrane réticulé, l'agarose réticulé, le charbon actif et un méthacrylate, qui est associé à l'oxydoréductase et qui, une fois la réaction terminée, est séparé de la composition de biotransformation et l'oxydoréductase associée à l'adsorbant est séparée par filtration, sédimentation ou centrifugation pour la réutilisation du mélange réactionnel.

2. Procédé selon la revendication 1, **caractérisé en ce que** les adsorbants sont choisis dans le groupe constitué par l'oxyde d'aluminium, le gel de silice, le silicate de Mg, la bentonite, la Celite^{®}, XAD, Dowex^{®}, Amberlite^{®}, Sepharose^{®}, Sephadex®, Superose®, la cellulose.

3. Procédé selon la revendication 2, **caractérisé en ce que** les adsorbants sont XAD, Florisil^{®}, le gel de silice ou la Celite^{®}.

4. Procédé selon la revendication 3, **caractérisé en ce que** l'adsorbant est la Celite^{®}.

5. Procédé selon l'une quelconque des revendications 1 à 4, **caractérisé en ce que** l'alcool secondaire chiral est un composé de formule (II) ou (III) 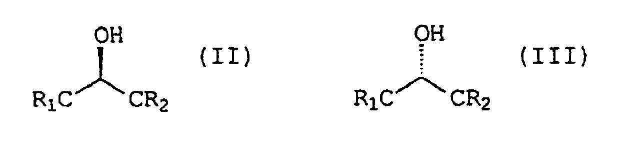 où R₁ et R₂ sont différents l'un de l'autre et représentent un radical organique.

6. Procédé selon la revendication 5, **caractérisé en ce que** R₁ et R₂ sont des radicaux organiques différents ayant de 1 à 20 atomes de carbone, un ou plusieurs atomes de carbone des radicaux R₁ et R₂ pouvant être remplacés par des atomes choisis dans le groupe constitué par B, N, O, Si, P et S ou pouvant être substitués par F, Cl, Br, I, par des groupes cycloalkyle en C₃-C₈, aryle en C₆-C₂₀, hétéroaryle en C₅-C₂₀, éventuellement substitués, par des radicaux silyle ainsi que par CN, NH₂, NO ou NO₂.

7. Procédé selon l'une quelconque des revendications 1 à 6, **caractérisé en ce que** l'oxydoréductase est une carbonylréductase à spécificité pour S ou R.

8. Procédé selon l'une quelconque des revendications 1 à 7, **caractérisé en ce que** la composition de biotransformation comprend un cofacteur redox choisi parmi des composés du groupe constitué par NAD, NADP, NADH, NADPH et leurs sels.

9. Procédé selon la revendication 7 ou 8, **caractérisé en ce que** la carbonylréductase est un alcool déshydrogénase et le co-substrat est un alcool.

10. Procédé selon la revendication 9, **caractérisé en ce que** l'alcool est l'isopropanol ou le 2-butanol.

11. Procédé selon les revendications 1 à 10, **caractérisé en ce que** les produits de départ de formule générale (I) sont convertis à raison de > 80 % en un alcool secondaire chiral.

12. Procédé selon les revendications 1 à 11, **caractérisé en ce que** l'adsorbant séparé qui est associé à la carbonylréductase est introduit dans une composition de biotransformation selon la revendication 1, aucune carbonylréductase n'étant ajoutée à la composition de biotransformation ou seule une quantité de carbonylréductase équivalente à celle qui a été perdue lors de la séparation de l'adsorbant étant ajoutée à la composition de biotransformation et ce deuxième cycle de réaction étant effectué comme dans la revendication 1.

13. Procédé selon la revendication 12, **caractérisé en ce qu'**on effectue jusqu'à 20 cycles de réaction en utilisant l'adsorbant séparé dans le cycle de réaction précédent.

14. Procédé selon les revendications 1 à 13, **caractérisé en ce que** l'alcool secondaire chiral est extrait du mélange réactionnel, après séparation de l'absorbant, par distillation ou au moyen d'un solvant organique non miscible à l'eau.
